# EUROPEAN PATENT APPLICATION

(11) **EP 1 349 082 A1**
(43) Date of publication of application: **01.10.2003**
(21) Application number: 02007419.1
(22) Date of filing: 28.03.2002
(51) Int. Cl.: G06F 17/30

(54) **Method and apparatus for querying relational databases**

(71) Applicant: LION bioscience AG, 69123 Heidelberg (DE)
(72) Inventor: Etzold, Thure, Cambridge CB1 2DZ (GB); Luo, Jie, Cambridge CB1 3RX (GB); Seers, John, Norwich NR2 3BW (GB)
(74) Representative: Schohe, Stefan

(57) **Abstract**

The invention provides a method of evaluating a query involving a relational database comprising a relational database management system (RDBMS), said query relating to at least one table of said relational database, said method comprising determining a table as a gateway table for evaluating said query, retrieving one or more unique identifiers of said gateway table related to one or more entries in a table to be queried, retrieving information from tables to be queried related to said retrieved unique identifiers of said gateway table, and providing a result set comprising the retrieved primary keys of the gateway table in relation to said retrieved information. The invention further provides a related data processing system and program.

## Description

The present invention relates to a method for evaluating a query involving a relational database and a related apparatus and a program.

A major goal in bioinformatics is to provide search tools with which biologic information can be retrieved quickly, effectively and completely. Presently there are huge databases storing biological data. Apart from that there are, however, a number of smaller databases and also electronic data which are not structured like databases, but rather as flat files, e.g. publications in scientific journals. Thus search tools in bioinformatics frequently have to combine data from various sources. Since the user wishes to have the result almost immediately on the screen, time is of essence in this regard.

The SRS query language and package has proved to be a powerful instrument in building such search tools. SRS allows to combine information from various sources. Principles of SRS are set out in WO00/41094. Basically, SRS works in a two-step process. In a first step entries in a data source are identified and in a second step extracted by a parser. Whereas this concept works well with flat files, the application to relational databases leads into problems as the extraction of information related to an identifier, e.g. a key of a table, may take a comparatively long time in a large database. This is due to the fact that the required information usually has to be collected from a plurality of different tables which may not be directly linked to each other. Current relational database management systems work in the way that they establish a joined table for all tables involved in a query. Since the size of the joined table is essentially the product of the sizes of the rows of the individual tables, this quickly leads to large result sets and, accordingly, to long processing times for evaluating these result sets.

It is an object of the present invention to provide a method, apparatus and program for performing queries in a relational database wherein queries can be handled more easily and are easier to understand and wherein queries can be processed more quickly and especially to provide a method which is compatible to the process of extracting information from other data sources, such as flat files.

According to the invention this object is accomplished by a method of evaluating a query involving a relational database comprising at least one relational database management system (RDBMS), said query relating to at least one table of said relational database, said method comprising determining a table of said relational database as a gateway table for evaluating said query, retrieving by means of said RDBMS one or more unique identifiers of said gateway table related to one or more entries in a table to be queried, using said RDBMS, retrieving information from one or more tables to be queried related to said retrieved unique identifiers of said gateway table and providing a result to said query. This result may be a result set in the conventional sense of a result table, comprising the retrieved primary keys of the gateway table in relation to said retrieved information, or an object comprising the result of the query, which may be derived from such a conventional result table.

According to the present invention, a "gateway table" is chosen which forms the starting point to the evaluation of the query. In a way it serves as the entry point or "gateway" to the evaluation of the query. In accordance with the invention, either prior to the submission of said query or during the evaluation thereof, a table is designated as a gateway table, meaning that the step of retrieving one or more unique identifiers is performed with regard to this table, provided, of course that this table is related to tables referred to in the query. The designation of a table as a gateway table may be done in advance, e.g. as part of the system or database settings or by means of user settings prior to the submission of the query. The invention may, however, also provide that during the process of the evaluation of the query, a table is determined as a gateway table according to suitable criteria and that the above-mentioned steps are carried out with regard to this table subsequently. In the preferred embodiment, the unique identifiers referred to above are primary keys or unique indices of the gateway table. The invention may, however, also provide that said unique identifier is a combination of indices of different columns and, in certain instances, the combination of all indices of a row of the table, which is, per definition, a unique identifier in relational database management systems.

The invention may provide that when retrieving said one or more unique identifiers of said gateway table, in a first step a predetermined index or predetermined indices are retrieved. Should it turn out that this index or these indices do not specify a row of the table in a unique manner, a unique identifier is created from such indices according to a suitable procedure. One procedure may be to add indices to the indices retrieved in said initial step until the combination of said indices is a unique identifier for a row related to an entry in a table to be queried. Given the case, this may be continued until all indices of a row are comprised in the combination. Having thus created a unique identifier, it is verified that the related row still relates to said entry in a table to be queried. If it does not, the respective identifier is discarded. Alternatively, one may, for example provide that if the indices retrieved in said initial step actually designate a plurality of rows of the gateway table, one only proceeds with the first row, thereby accepting a potential loss of information.

The invention may provide that said relational database comprises one or more predetermined hub tables, and said query relates to at least one table of said relational database and wherein said method comprises retrieving by means of said RDBMS one or more unique identifiers of a hub table related to one or more entries in a table to be queried, using said RDBMS, retrieving information from tables to be queried related to said retrieved unique identifiers of said hub table and providing a result to the query, e.g. as a result set (object or result table) comprising the retrieved primary keys of the hub in relation to said retrieved information.

The invention may provide that one or more libraries are defined on one or more databases. In the sense as used herein a library is defined as a collection of tables which are linked to each other and which are not necessarily within the same database, wherein there is exactly one table defined as a hub table. All tables in a library are linked directly or indirectly to the hub. Therefore, any entry in a library can be accessed through an entry in the hub and the (direct or indirect) relation to the hub. Thus, the hub table can be considered as representing the library. That is, a library in the sense of the invention always has one unique entry point or gateway for the evaluation of a query, namely said single hub. If a library is exclusively defined on one database, it may be viewed as a restricted database. On the other hand, if tables of a second database are comprised in a library, the library is, in a way, an extension of a database. Different libraries may share the same tables. Using the concept of libraries according to the present invention, it is possible to define concepts at a higher level than the underlying databases which can be tailored to the needs of the application or the wishes of the user without affecting the underlying structure of the databases.

Using the concept of libraries, it is apparent that a query in a plurality of databases can be carried out without having a hub in each of the databases, if the library extends over a plurality of databases. In this case there is only one hub in one database, namely the hub of the library (which is, by definition, comprised in one of said databases).

Thus, whenever reference is made to a hub or gateway table of a database, it should be understood that this hub or gateway table may be a hub or gateway table of a library defined on said database or on a plurality of databases comprising said database. Since a library is essentially a database structure superimposed on the underlying databases, it should be understood that whenever reference is made to a query in a database or the evaluation of a query in a database, this reads mutatis mutandis on queries in a library, unless an indication to the contrary is given.

A hub table (also called "hub" subsequently) is essentially a predetermined gateway table for evaluating queries in said database or library. As a rule, a query is for a complete set of related entries in a searchable entity, i.e. all information in any table that is related to a uniquely identified entry in a table, or a part of such a set. If the searchable entity is a relational database, said complete set of related entries comprises all entries directly or indirectly linked to said entry in said database. By defining a higher level entity on databases or across databases, such as a library (to be understood in the above-mentioned sense), a complete set of related entries is a set of all entries in said library that are directly or indirectly related to a uniquely identified entry in a table comprised in said library. In many instances, it is neither desired nor necessary to provide all information related to a certain entry in a table. In such cases one will restrict the query to certain columns of tables or certain tables. In the SQL language, such selection can be made using standard syntax. In a preferred embodiment, the selection of tables and/or columns of tables will be predetermined by a user or administrator as part of the settings of the user interface prior to typing in a specific query. If a library is defined exclusively on one single database, a complete set of related entries in the library will be part of a complete set of related entries of the whole database. This means that by defining the library on the database a restriction as to the tables and columns of tables that are to be queried has been made so that when a query referring to this library is submitted no other tables of the database will be queried. It should, however, be noted that a complete set of related entries in a library may not be completely contained in a complete set of related entries of a database, since the library may comprise tables of two or more different databases.

In most instances the query input typed in by a user will consist of query conditions specifying data to be comprised in the result of the query. For the purpose of this discussion a query condition will be considered as a condition in the query that certain elements are to be present in certain specified data fields. With regard to a relational database this would mean that certain entries in certain tables have a specified value. For a flat file this would mean that a certain sequence of data, e.g. sequence of letters, is found in a certain data field. Although this is what the user sees, the query conditions do not form the entire query that is processed by the system. In order to return a result, the system needs additional information on which information related to said query conditions shall be returned, e.g. related information in other tables. This additional information is contained in settings which may partly or as a whole be determined by a user. The system will generate on the basis of these query conditions and the predetermined settings regarding the tables and columns to be queried one or more query commands to be submitted to the RDBMS. The query conditions usually impose a condition on the rows of the columns to be selected. In such instances, the method according to the invention will first look for entries that satisfy the conditions expressed in the query conditions and then return one or more hub indices or, in the preferred embodiment, unique identifiers of the hub table.

The invention may also provide that all entries in a column are to be retrieved in the query, in which case the query would retrieve all unique identifiers of the hub table related to a certain column.

The invention may provide that said query is for complete sets of related entries of one or more databases or libraries or predetermined parts of such complete sets of said relational database and comprises one or more query conditions related to said database or library, wherein said method comprises:
- identifying a gateway table related to entries specified in a query condition,
- identifying one or more unique identifiers of said gateway table related to said entries conforming to query conditions,
- retrieving complete sets of related entries or parts thereof which are related to said unique identifiers of said gateway table.

In many instances a query will relate to different entities, e.g. to two databases or two libraries, a database or a library and a flat file or multiple databases, libraries and/or flat files. Likewise it may be that a query relates to tables in one database which are related to different hubs of the same database. In all such instances the invention provides, according to a preferred embodiment thereof, that those parts of a query that are related to a hub or, in case of an entity other than a library or a relational database, to one or more sub-entities of said second entity are processed separately and the result of the partial searches are combined by using relations between the unique identifiers of the hubs, e.g. primary keys of the hubs and identifiers of other entities and, given the case, relations between a plurality of such identifiers. In a simple case, it is determined which unique identifier of a hub relates to which other unique identifier of another hub or to which identifier and the results of the related two partial queries are combined.

The invention may provide that said query involves at least a second searchable entity outside said database or outside a library involved in said query, said second entity comprising sub-entities each having at least one identifier uniquely identifying said sub-entities, and wherein said method comprises:
- retrieving one or more identifiers of sub-entities of said second searchable entity related to said query, especially related to query conditions of said query,
- retrieving one or more unique identifiers, e.g. primary keys, of a hub of said relational database or library related to said retrieved identifiers of said sub-entities,
- retrieving sets of related entries, especially complete sets of related entries, related to said retrieved unique identifiers of said hub,
- retrieving information from a sub-entity identified by a retrieved identifier in said second entity,
- combining the retrieved information from said second searchable entity and said data base or library into a result set.

Additionally or alternatively the invention may provide that said query involves at least a second searchable entity outside said database or outside a library involved in said query and comprising sub-entities, each sub-entity having at least one identifier uniquely specifying said sub-entity, and wherein said method comprises:
- retrieving one or more unique identifier of a hub of said database or library related to entries related to said query, especially related to query conditions of said query,
- retrieving identifiers of sub-entities of said second searchable entity related to said retrieved unique identifiers of said hub,
- retrieving sets of related entries or parts thereof related to said retrieved unique identifiers of said hub,
- retrieving information from said sub-entities identified by identifiers retrieved in said second searchable entity,
- combining the retrieved information from said second entity and said data base or library into a result.

A searchable entity in the sense of this application may be a database, a library and identifiers thereof may be primary keys or other unique identifiers of a hub table.

The invention may provide that said second searchable entity is a second relational database or a library and said identifier is a primary key or another unique identifier of a hub table in said second relational database or second library, said sub-entity being a table, a combination of linked tables or a part thereof.

The invention may provide that said second searchable entity is a collection of flat files with the sub-entities being flat files in this collection.

The step of retrieving information related to the retrieved identifier of the second entity and/or retrieved unique identifier of the hub may be performed prior or after the step of retrieving relations between the identifier of the second entity and a unique identifier or identifiers of the hub or hubs. In the preferred embodiment of the invention, one proceeds, however, by first evaluating the identifiers of one entity related to a query condition and then retrieves related identifiers of the other entity and uses these identifiers of the other entity as the starting point for retrieving the related information in said other searchable entity. If the query conditions relate both to the database or library and to the second searchable entity, one preferentially proceeds by first retrieving the identifiers of the second entity and the unique identifiers of the hub or hubs related to the query conditions, then establishing combinations of identifiers of said second entity and identifiers of said hubs which are related to all query conditions, i.e. both the conditions relating to the database or library and to the second searchable entity, and then retrieving additional information only for those combinations which are consistent with all query conditions.

The invention may provide that said step of retrieving a relation between identifiers of said second searchable entity and unique identifiers of hubs of said database or library comprises the step of discarding combinations of unique identifiers of hubs and identifiers of said second searchable entity which are not consistent with the query conditions and, retrieving only such additional information related to an identifier which is comprised in combinations of identifiers consistent with the selection parameters.

The invention may provide that the query relates to tables related to at least two hub tables, wherein said method comprises:
- retrieving one or more unique identifiers of a hub table or hub tables related to entries satisfying query conditions in tables related to the respective hub,
- retrieving unique identifiers of the respective other hub or hubs related to said retrieved unique identifier related to entries satisfying said query conditions,
- retrieving sets of related entries or predetermined parts thereof related to said retrieved unique identifiers of said hubs according to the query,
- combining the retrieved information related to said hubs into a result.

Retrieving unique identifiers of the respective other hub or hubs may involve unique identifiers which have been found in the first step of retrieving unique identifiers related to query conditions. In such a case, a consistency check is carried out whether the combination of unique identifiers meets the query conditions.

The invention may provide that said step of retrieving a relation between a unique identifier of said hub tables comprises the step of discarding combinations of unique identifiers of hub tables which are not consistent with the query conditions and retrieving only such additional information related to at least one unique identifier of at least one hub which is comprised in a combination of unique identifiers consistent with the search parameters.

Of the two above-mentioned hubs, at least one may be the hub of the library or both of them might be hubs of a library. It may also be provided that said two hubs are hubs within the same relational database. These can, but do not have to be hubs of two libraries defined on this database. One or both may also be a hub which is not related to a library.

The invention may also provide that after performing a partial query for each hub for said sets of related entries or parts thereof, the respective results are joined and subsequently checked for consistency with the query conditions and, given the case, for redundancies. The invention may also provide that an object is created for each partial result and these objects are further processed to yield the result of the query.

The invention may provide that the step of retrieving identifiers of a searchable entity which are related to another identifier of a searchable entity is performed on the basis of pre-established relations between identifiers of said entities.

The invention may also provide that the step of retrieving identifiers of a searchable entity which are related to another identifier of a searchable entity is performed dynamically during the execution of the query. The invention may also provide that said step is performed partly on the basis of pre-established relations and partly dynamically.

Especially, the relation between hubs of a database and an identifier of another searchable entity can be established on the basis of a static link or of a dynamic link that is created "on the fly".

Retrieving information from tables referred to in the query and related to a unique identifier of a hub may be a comparatively slow process for large databases which is due to the way current relational databases work. If a query involving a plurality of tables is input under a standard language, e.g. SQL, the RDBMS will form a joined table of all tables involved which is essentially the cartesian product of all tables. The number of data sets in a joined table is essentially the product of the number of rows of the individual tables involved which means that the size of the joined table grows exponentially with every further table involved in the query. This leads to a rapid decrease of computer speed with the number of tables involved.

According to one aspect of the invention this is counteracted by controlling more precisely what tables are involved. For the purpose of the further discussion it is helpful to use the representation of a database as a node diagram. A node diagram is to be understood as a graphical representation of the database, wherein tables are represented as nodes and links between tables as lines between the nodes. A link between the tables may especially be a link through a foreign key, but is not restricted thereto. Using the concept of a node diagram, any relation between two tables of the database can be visualized as a path between two nodes which is either direct (in which case there is a direct link between the two tables) or which is indirect and passes one or more intermediate nodes. Using this concept it is possible to control the number of nodes involved in a query and to formulate the query in a way that a defined number of intermediate tables are involved.

The invention may provide that in performing said step of retrieving information related to a unique identifier of said gateway table, selected tables are queried which, in a graphical representation of the database wherein the tables are represented as nodes and links between the tables are represented as lines between the nodes, form a connected graph connecting the gateway table to tables referred to in the initial query.

A further improvement of this concept resides in that a large query can be split up into a number of intermediate queries which are more easily evaluated, because the joined tables involved are much smaller. Essentially, a link between two tables in said graph is replaced by a junction. A junction between two tables means that the two tables are not involved in the same query, but that the values of the link keys or another input to the link found in a previous query for one table are used as the input for the link keys in a query involving the second table.

The invention may provide that said step of querying tables on said graph comprises performing consecutive partial queries, wherein a result of a previous query is used as input for a later query, preferentially the next query along said graph, a first of said partial queries involving the gateway table and a query other than the first query relating to a table referred to in the initial query.

According to the invention the partial queries are structured such that only tables are involved in a partial query which are directly linked to another table in said query and that every table in said graph is involved in at least one query.

Thus, there is a complete chain of queries leading from a hub table to a table referred to in the query.

When the relation between the tables is a foreign key relation, the invention may provide that the result of said previous query comprises the value of a foreign key of a table involved in said later query and wherein said value of said foreign key is used as input for said later query.

For each partial query, the result is stored to be retrieved later on for combining the results of various partial queries to a result or partial result of the initial query. For the sake of clearness, the query from which the method according to the invention starts is termed here and thereafter as "initial query".

According to a preferred embodiment, the queries are non-overlapping in the sense that, apart form the input of a previous query, only tables are queried which are not queried in another partial query.

Splitting up a big query into a plurality of smaller queries the result of which is subsequently merged has the advantage that smaller queries return smaller result sets. Since consecutive tables along graph are directly linked, there is also a direct join between consecutive tables, meaning that the joined table of a single partial query is usually not excessively large. How many consecutive tables are encompassed by a partial query depends on the relation between the tables. Usually, one will determine the various partial queries in a manner that the result set is easy to handle and to be purged of redundancies.

The invention may provide that after each partial query a redundancy check is done on the result table. According to one embodiment of the invention, objects without redundancies are created on the basis of the result set of the partial queries.

The stored results may be further filtered or purged depending on the outcome of further partial queries. For example, if a first partial query returns the value of a key that later on turns out not to be related to a table referred to in the initial query, data in the object related to said result and comprising said key may be removed from the result set.

The invention may provide that each partial query involves a table or a plurality of tables linked to each other and wherein each partial query has as input previously established values of keys of a table, especially link keys. Said link keys link said table or one or more of said plurality of tables to another table not involved in said partial query.

Said values of link keys may have been found as the result of a previous partial query. The key values used as input may also be values of a key of a gateway table that have been determined in a previous step. The first partial query differs in that the gateway table is part of the query while values of its keys are used as input.

The invention may provide that said graph comprises at least one branch node having links to at least two other nodes and wherein tables referred to in the initial query are related to separate branches deriving from said branch node, wherein a partial query is carried out involving the table corresponding to said branch node (branch table) and wherein at least one partial query is carried out for one or more tables contained in each branch which has the result of the partial query involving the branch table as an input.

The respective consequent partial queries for each branch may have further consequential partial queries eventually involving the tables referred to in the initial query. The chain of queries for each branch can be evaluated consecutive or parallel to each other.

If one of the branches involves a table related to a necessary query condition, e.g. specified in a WHERE clause under SQL, one will preferentially evaluate this branch first and thereby retrieve those keys of the branch node which are actually related to the entries satisfying said necessary condition. In general these will be less than those keys which were retrieved when the branch node was "passed", i.e. involved in a partial query, for the first time. Accordingly the result of the first partial query involving the branch node will be reduced to only comprise such keys of the branch node. This reduced set of key values will then be used as input for one or more partial queries related to the other branch.

If two or more branches are related to necessary query conditions one may also proceed by evaluating each branch separately on the basis of the keys of said branch node evaluated previously. The evaluation of each branch will each return as a result those keys of the branch node which are related to an entry satisfying a necessary condition. In a consequent treatment of the related result only those entries will be retained which comprise keys of the branch node related to entries satisfying either condition.

In a way, a branch table is similar to a hub table in that multiple queries are derived therefrom. In fact, in most instances, the hub table will also be a branch table; a typical example would be a database where the diagrammatic representation of the database is star-like with the hub at the center of the star. Thus, the methods for evaluating the branches deriving from a branch node can be similar to the methods of evaluating distinct graphs deriving from the same hub. One should, however, bear in mind that a hub table and a branch table are defined differently. The difference in the definition of a hub table and a branch table is that the hub table is a predetermined entry point for evaluating a basic query and does not necessarily mark a branch, whereas a branch table may also be a table that is determined during the evaluation of an initial query.

A method according to the invention may comprise the steps of
- identifying the hub or hubs related to tables referred to in the initial query,
- determining in said graphical representation of said database, at least for one hub, preferably for all hubs, an optimum graph connecting said hub to all tables which are related to said hub and which are referred to in the initial query,
- performing a query on said optimum graph.

It may be provided that evaluating said query on said graph, consecutive partial queries involving tables which are consecutive to each other with regard to said optimum graph are performed.

The optimum graph referred to above is a graph determined by an optimisation algorithm. Optimization algorithms for finding an optimum graph connecting given points, such as Dijkstra's algorithm, are well known in the art. The graph is preferentially optimized with regard to the speed with which the sequence of partial queries can be resolved. One way of proceeding is to assign a weight to each link between nodes in the graph and to vary an initial graph until it is optimized with regard to the accumulated weights. This weight may be implemented as a metric and accordingly the search is for the "shortest" path according to this metric.

The concept of using a node diagram to customize the query process and the concept of splitting up a large query into a number of smaller consecutive queries, especially the concept of junctions, are not restricted to the evaluation of queries involving a predetermined hub table and a table related to said hub. In fact, this concept can be used independently of the concept of hubs, e.g. in that for a query involving a relational database a gateway table may be defined which forms the starting point for the evaluation of the query and from which a path or graph to the tables involved in the query is established and evaluated, as described above. This gateway table need not necessarily be static or predetermined, such as a hub, but it may also be chosen with respect to a specific query, even in the process of evaluating the query in a way that the graph connecting this gateway table and the tables referred to in the query is optimized with regard to the speed of the evaluation process, e.g. with algorithms as mentioned above.

For example, a further application of this concept may be the evaluation of links between two databases. According to an embodiment of the invention relations between two databases are always established through the hubs of the two databases. In order to establish such a relation an existing link between two tables of the two databases (hereinafter referred to as "linking tables") can be employed to provide a dynamic link between two hubs of the two databases. Starting from a hub table in the first database and a certain unique identifier of this hub, the linking table in the first database is found and the relation of said hub table to said linking table is established, e.g. using a node diagram of the database. The entries in said linking table related to said unique identifier of the hub table and the related entries in the linking table in the second database are determined. Subsequently, the unique identifier of the hub of the second database related to the entries of said second linking table are determined. Although this procedure could be done in advance e.g. for all primary keys of the hubs involved, wherein relations between primary keys of hubs thus found could be statically stored in advance, it is presently preferred to perform these steps dynamically during the evaluation of a query. The concept of graphs in a node diagram and of junctions can be applied to dynamically execute such a link. Like it was explained before, a graph connecting the two hubs through the linking tables may be established (wherein the link between the two linking tables of different databases is treated like a normal link in a database) and a number of consecutive queries, starting with certain values of the primary key or another unique identifier of the first hub, eventually leading to the second hub, is performed and evaluated. For the sake of completeness it should be mentioned that one could also use a mixed type of link, where part of the steps are performed in advance and part of them dynamically.

The invention may provide that said step of retrieving unique identifier of said gateway table comprises:
- determining a table that is referred to in the initial query,
- determining, in a graphical representation of said database, wherein tables are represented as nodes and links between tables as lines between the nodes, a gateway table connected to said table,
- querying said database for one or more indices, especially primary keys, of the gateway table which are related to said table.

The invention may provide that one or more specific entries of said table are implied by a query condition and said database is queried for one or more indices of said gateway table which are related to said entry.

The invention may provide that in said graphical representation, a path from said table to said gateway table is established and said query for said indices is performed by querying all tables corresponding to nodes in said graph for the values of link keys between the tables in said graph, starting from the table referred to in the query and, given the case, certain entries thereof.

The invention may provide that said path is selected as a shortest path between said table and said gateway table according to a predetermined metric.

The invention may provide that said path is part of or identical to the graph for determining partial queries for retrieving additional information from tables related to said gateway table.

The invention may provide that, if an index or a group of indexes related to the same row of the gateway table and determined by said step of querying the database does not uniquely identify a row of said gateway table, a unique identifier for one or more rows of the gateway tables is determined that is related to said indices.

The invention may provide that partial queries used for evaluating the initial query are at least partially and preferable completely created dynamically during the process of said evaluation.

The invention may provide that said result set is represented in an object oriented representation.

The invention may provide that the result of said initial query is expressed as an object derived by object-relational mapping. In the preferred embodiment, after each partial query performed in the process of evaluation of the initial query, an object is created by object-relational mapping and the object representing the result of said initial query is derived from these objects relating to the partial queries. In one embodiment this object or objects created from the result of a partial query are represented in XML.

The invention may provide that said evaluation of said query is performed under the control of an object manager, said object manager comprising a sequence of commands to be executed by a computer system.

The invention may provide that said object manager handles an object which represents the schema or part of a schema of one or more databases to be queried.

The invention may provide that said object manager defines classes which are dynamically created and initiated.

The invention may also provide a data processing system for controlling the evaluation of a query involving a relational database comprising a relational database management system (RDBMS), said query relating to at least one table of said relational database, comprising:
- means for determining a table as a gateway table for evaluating said query,
- means for establishing a relation between a table or tables to be queried and a gateway table,
- means for causing the RDBMS to retrieve one or more unique identifiers of said gateway table related to one or more entries in a table to be queried,
- means for causing the RDBMS to retrieve information from tables to be queried related to said unique identifiers of said entry,
- means for providing or causing to be provided a result to said query, which may be a result set or result object comprising the retrieved primary keys of the gateway table in relation to said retrieved information.

Said data processing system may comprise means for setting certain tables in said relational database as predetermined gateway tables for queries to be evaluated.

A data processing system according to the invention may, in further embodiments, comprise means for controlling the execution of a method as previously described by a data processing system or data processing systems.

The invention also provides a computer program causing a computer or computer system, when executed thereon, to perform the steps of a method as previously described and a computer readable storage medium, comprising such a program.

According to an important aspect of the invention, the method according to the present invention uses a standard relational database management system and defines all manipulations of queries outside this relational database management system. This allows for a great extent of flexibility and in fact creates a platform that can be used independently of the underlying system. Surprisingly, it was found that according to the method of the present invention, especially evaluating a query according to the concept of graphs and junctions, may lead to a significant increase in speed, which can be as high as 50 %, and even higher, if tables with a plurality of 1:N relations are involved so that combinatorial explosion sets in.

Further features and advantages of other inventions will be apparent from the following description of an embodiment of the invention, taken in conjunction with the drawing.
- Fig. 1: shows a node diagram of a simple database referred to in the explanation of a specific example.

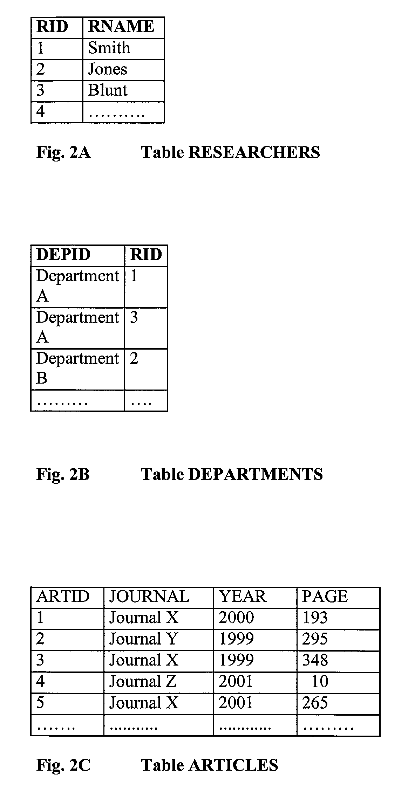

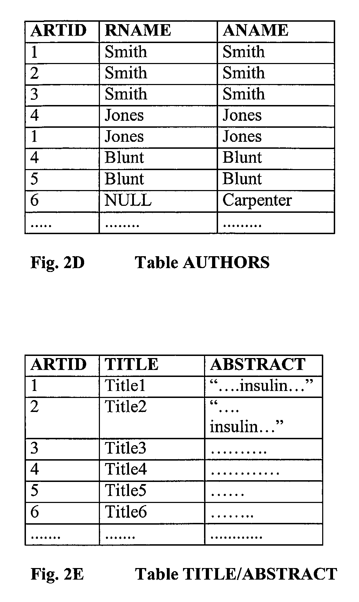

- Figs. 2A to 2E: show the tables of the database according to Fig.1
- Fig. 3: shows a node diagram for a second example.

According to one embodiment of the invention one first performs an analysis of the database for which the invention shall be implemented. Essentially one collects information about the tables of the database, the columns, their type, their size, their indices and keys. Analysis tools for collecting such information are readily available in the art. For example JDBC (Java Database Connectivity) or any other RDBMS API may be used to collect such information. From this information, a node diagram of the database is constructed in the form of a minimum spanning tree using the key relationships between tables. Essentially, for any table one looks for a key linking this table to another table (link key) and if there is such a link key, one introduces an edge between two nodes representing the two tables. It should be understood that of course one does not draw an actual graph, but rather constructs an object structure which maps to such a graph. For ease of explanation, reference is made herein to the graph or elements of the graph, it being understood that such reference is meant to relate to respective elements, relations or operations on the corresponding data structure.

Usually, both nodes and edges will be assigned some attributes. For example, one may assign a weight to each edge which is used later on for determining the optimum graph, one may assign an attribute indicating that the edge represents a 1:1, 1:N, N:N or N:1 relationship or one could assign attributes representing the presence of indices in particular relations between two tables, just to mention a few examples. Attributes assigned to nodes (tables) may, for example, be the name of the columns, their size, or other relevant information.

At this point one may also add or change relationships between tables manually where a relationship is required. For example, if there is a table listing the authors of a scientific publication and a table relating to contact details, such as e-mail addresses, which table is not or not directly related to the author table, one may manually insert a relation between the name of the author in the one table and his or her e-mail address in the other table. This relation may be implemented as a modification of the database. It may, however, also be implemented outside the database as an external link between the two tables.

The result of this step is an object representing the schema of tables. This schema object is stored for further use. The exemplary embodiment described here uses the SRS environment and under this environment this schema object will be stored as an SRS object manager object, for example through the SRS Java Application Programming Interface (API). The object manager is a tool controlling the evaluation of a query under SRS. Details of the SRS query language can, for example, be found at http://srs.ebi.ac.uk.

As a further step, hub tables are defined which shall serve as entry points or gateways to the tables in the evaluation of a query. A requirement for a hub is that it comprises unique identifiers, i.e. every data set in the table can be uniquely identified by an identifier. Preferably, unique indices or primary keys are used as unique identifiers, but e.g. unique combinations of indices may also be used. There are essentially two considerations that can lead to defining a table as a hub. One derives from the user sphere and seeks to provide a focus point in the query that is instructive to the user. The other consideration is more technical and seeks to define the table in a manner that queries can be carried out most efficiently. Thus, the hub table may be a table representing information that is a central point of interest. From a technical point of view, the hub table should be a table that is likely to be queried and which is preferentially linked directly to other tables which are likely to be queried or related to such table in a way that queries involving the hub and such tables can be evaluated most quickly by the RDBMS. One may, for example, think of establishing statistics which tables are most frequently queried in a relational database and which combinations of tables are most frequently queried and choosing the hub table accordingly. The two considerations can frequently be reconciled to each other in that a table that is frequently queried is usually also a table of primary interest to a user.

The focus of interest may shift, according to the user. Whereas one user may be primarily interested in authors of scientific applications, another user may primarily be interested in certain keywords in a scientific publication and not or only mildly interested in the author. Accordingly, the invention may provide that a user or a database manager can define a hub or hubs as part of the user settings or even when typing a query. It should be noted that more than one hub may be defined in a single database. This may be useful in large databases in that it allows smaller sub-structures, such as libraries, to be formed which can be used to increase performance in the case of particularly large schemas containing large data sets. Another reason that can lead to defining a plurality of hubs in a database is that one can in this way define clusters of tables in a database which are known to be queried in conjunction or, as far as tables of different clusters are concerned, are known not to be frequently queried in conjunction with each other. This will later on restrict the number of possible paths between a hub and a table to be queried in many instances and thus contribute to the speed of the application. The definition of hubs has a still further purpose under SRS. By choosing a hub table and unique identifiers, e.g. primary keys, as identifiers for entry points to the database, a data structure is achieved that can be viewed as centered around a "hub column", meaning that access to the data is through a well-defined identifier. This establishes a similarity to flat files and accordingly allows to use procedures of SRS for relational databases which have been developed for flat files.

When a user inputs a query, he usually only specifies keywords he is interested in and gets back all information related to said keywords. These may be complete database entries. An embodiment of the invention may provide that the user can specify in the graphical user interface which tables or columns of a database he is interested in. This can, for example, be implemented by providing tick-boxes which the user uses for indicating the information of interest. Additional information about the extent of the requested information may be implemented in the system settings which may or may not be changeable by a user.

When a user types in a query, the system, according to this example, works in a two-step process, in analogy to the standard procedure under SRS for retrieving information. In the first step unique identifiers of a hub (or the hubs) are identified which relate to the tables relating to the query conditions, e.g. the tables containing the keywords requested by the user. In a second step, all information is retrieved that relates to the query conditions, to the extent such information has been specified in the system settings or by a user input.

More precisely, in the first step an analysis of the query is carried out with a parser, e.g. with an ICARUS parser. The result of this analysis could be a binary tree representing a hierarchical analysis of the query and specifying at a higher level the database, at a lower level the identifier field, e.g. "author" and at an even lower level the keyword required, e.g. "Smith". The system now maps this query to the node graph of the database stored in the system. To stay with the above-mentioned example, the system verifies whether there is a table in the specific database uniquely identifying authors and the column in which the name of the author should occur. If such a table is found, the system verifies whether and which hub is related to this table in said node diagram. It then generates a query for unique identifiers, e.g. primary keys, of the hub table related to the entry in said table meeting the query conditions. Since it has been previously established that the hub is related to the tables in the input query by at least one path, there should be at least one unique identifier of the hub related to the entry.

In a specific embodiment, the system establishes a path in said node diagram from the table to be queried to the hub table and, in querying the unique identifier of the hub, also queries for the keys of the intermediate tables (nodes) in the path related to the entry specified by the query condition. In this way, a complete set of relations to the entries meeting the query condition, including the unique identifier of the hub, is established.

Assuming that there is only one hub and one database involved in the query, the system now establishes a graph or tree in said node diagram having the hub table as its origin wherein all tables referred to in the query appear as nodes. This graph is preferably a graph without any loops, i.e. there is only one path along the graph from the hub table to any table referred to in the query. The graph may have several branches originating in the hub and each branch may contain further branches. Ideally, the part of the graph between tables referred to in the query should be minimal. This is, however, not to be understood in a graphical sense, but rather in the sense of a metric defined on the graph and used for the optimization algorithm. For example, the evaluation of a query corresponding to a graph comprising a plurality of intermediate tables with 1:1 relations between them may be faster than the evaluation of a query corresponding to a graph with one single intermediate table having a 1:N relation to its neighboring tables along the graph and thus be "shorter" in the sense of a related metric.

Having established the optimum graph, the graph is evaluated in that a query is made for all entries in tables referred to in the initial query that are related to the values of the unique identifier identified in the first step. More specifically, the system creates an object plan for the query that consists of classes and partial SQL queries which are created dynamically. The information necessary for creating and instantiating classes and partial queries is held in a data structure called "turntable" which contains all the information needed to execute a query with different initial inputs, i.e. different values for the hub identifiers. The object plan is created according to the schema and information passed as to the graph for this query and may involve the optimization step. Once this object plan has been created, it is given initial information, i.e. the values of unique identifiers of the hub and subsequently run. In evaluation of the query, the system will query for the value of all entries of the tables represented by nodes on the branch which are related to the primary key of the hub. As pointed out previously, for a large number of tables along a branch this can, if done in the usual way, lead to large result sets with a lot of spurious entries. According to the invention, the query involving the tables of a partial graph along a branch is split up into a plurality of consecutive queries of tables along the graph, wherein the respective numbers of tables involved in the partial query is smaller than the number of tables in the query that would involve the entire branch. These multiple queries are sequential to each other in that the result of prior queries is stored and at least partly used as an input for at least one subsequent query.

The technique of connecting two consequent queries along a graph in that the result or part of the result of the first query is used as input to a consequent query, is referred to as a junction herein. A junction means that two tables corresponding to nodes in the graph which are directly connected to each other are not involved in the same query. For each pair of tables corresponding to directly connected nodes along the graph, there is a link, usually a link key, establishing the relation between the two tables. Using a junction, input to the link, usually the values of the link key established in the evaluation of a previous query involving one of the tables will be used as the input for a subsequent query involving the other table. This means that there will be no join involving these two tables and, accordingly, the result sets of each partial query will be smaller.

After each partial query it is determined whether a key determined in a previous step is related to a subsequent table along the graph at all. If it is not, the respective key value will be removed from the result of the partial query. Additionally, filtering and a check for redundancies and/or consistency may be carried out for the result of each partial query prior to storing. A redundancy check may, in one embodiment, start from the columns relating to the tables along the part of the graph currently queried which are most distant to the hub. If an identical combination of keys turns out at this level and there is no N:1 or N:N relation along this part of the graph, it means that there is a redundancy and the respective entry is removed. Other techniques of redundancy checking may, of course, be applied. Since only a small result set is to be created and filtered in each step, such filtering, redundancy checking etc. may be done more quickly and efficiently than the redundancy check and filtering of a large result set which would result if all tables referred to in the initial query were queried at once. The result of each partial query, having redundancies and/or inconsistencies removed, is stored as an object and information is stored that is needed to retrieve said object.

If, in evaluation of a branch of the graph, a further branch is identified, subsequent queries along each branch may be carried out separately, either parallel or consequential to each other. If the subsequent branches are relatively short, one may, however, also choose to involve the tables along these subsequent branches, given the case, together with the table corresponding to the branch node, in one single partial query.

Having evaluated a branch entirely in this manner, the results of the partial queries are combined and again checked for redundancy and consistency. Having evaluated each branch originating from a hub, one has obtained a result set for each branch, each identified by the value of the unique identifier of the hub. The results for different branches are then merged and again checked for consistency and redundancies to obtain the final result.

This result is expressed as an object structure, e.g. an object structure as is provided in the framework of SRS. Defining this object structure or objects representing results of partial queries, one can make use of dynamic classes, i.e. classes that are defined while processing and then instantiated to create objects. Dynamic classes per se are not new and have been known in the language Smalltalk, but are not available in current languages such as C⁺⁺ or Java which have only static or compile time classes. SRS supports dynamic classes, although previous applications did not make use thereof.

According to an important aspect of this embodiment, all queries created during the evaluation of the initial query are dynamically created while processing. In other embodiments, partial queries along certain partial graphs between hubs and tables which are frequently asked for may be determined in advance and optimised. It can also be contemplated to determine certain partial routes leading to a "cluster" of tables in advance, wherein the final part of the route to the required table is dynamically determined ("on the fly"). A further optimisation technique may be disregarding some connections in the graph of a database.

The above-mentioned two-step process can be viewed as resolving the query conditions in a first step and retrieving information related to the result thereof in a second step. In one embodiment of the invention the tables comprising the entries which meet the requirements of the query conditions are not necessarily part of the partial queries performed in said second step, e.g. if the information to be extracted from the table is restricted to the value of the entries relating to the query condition. It may also be provided that already in the first step all information related to the entries specified by the query conditions in the same table is extracted and stored so that there is no need to "revisit" the related table later on.

The above-mentioned two-step process is not necessary. One may imagine an embodiment wherein the system retrieves additional information when retrieving the unique identifiers of the hub, starting from the table referred to in the query condition. One may, for example, provide that in querying for the primary keys of the hub, the system queries also for the keys of the intermediate tables in a path starting from the entry specified by the query condition to the related hub and, in subsequent queries, retrieves information related to these keys.

Previously, a description was made for the case that only one hub or only one database or library is involved.

Cases where there is a plurality of hubs involved, e.g. in cases of a plurality of databases, a plurality of libraries or a plurality of hubs in one single database, are treated in such a manner that the initial query is split up into a number of partial queries, part of them corresponding to graphs having hubs as end points and the remaining part of them containing only one hub.

In the case of two hubs, one may, in a first step, retrieve the values of the unique identifiers of the hub tables related to tables which have entries meeting a query condition, as described above, said relation to said tables not involving the respective other hub. Subsequently and in a second step, one will determine an optimum graph which consists of partial graphs (corresponding to a partial query) related exclusively to one single hub and not involving any other hub and one or more partial graph (corresponding to one or more further partial queries) connecting the hubs to each other. One will then evaluate for each hub the partial graph exclusively related to this hub and store the respective results. One will then evaluate the query or queries corresponding to the partial graph or graphs between the hubs. The result of these partial graphs is then combined to yield the result for the entire query.

Alternatively, one could apply the concept of junctions for evaluating the link between the two hubs and introduce a junction behind each hub in the partial graphs connecting two junctions and, given the case, at further locations in these graphs. Evaluating these partial graphs linking the hubs, one starts with the values of a unique identifier of one hub and evaluates a query for related values of unique identifiers of the other hubs, which can be done in the same manner, especially using junctions, as was described before for the case of the evaluation of a query involving a hub and related tables. As a result, one will obtain a set of values of the unique identifiers of the other hubs which is then checked for consistency with the query conditions. This way of proceeding is especially advantageous if the query conditions only relate to tables related to one hub. In this case, one will only retrieve primary keys of this hub in the first step and the other hub is effectively treated like a branch point in a graph with a junction introduced instead of the last link on the graph connecting to the second hub.

Depending on the nature of the hubs and on whether the hubs are predefined in the system setting or chosen by a user, one may contemplate to store predetermined relations between the primary keys of a plurality of hubs in one single database. Conceptually, this would mean that one introduces in the node diagram a direct link between the two hubs which would be used instead of the dynamic link described above.

Another possibility that may be contemplated is retrieving the primary keys of all hubs in one query involving all query conditions in the first step of the procedure, thereby obtaining the relevant combinations of primary keys. This may be a sensible way of proceeding especially in cases where there is a direct link, especially a 1:1 link, between the hubs.

Cases where more than one database is involved can be treated in a manner similar to the case of a plurality of hubs. Assuming, for the sake of simplicity, that each database comprises only one hub and that the databases are linked to each other through linking tables, one in each database, one can consider the link between the two databases through the two linking tables as a normal edge in a node diagram. Representing the two databases in such a manner, there is conceptually and as far as the method of evaluating a query according to the present invention is concerned, little difference to the case of two hubs in a single database. That is, after retrieving the related unique identifiers of the hubs on the basis of the query conditions, as described before, one will determine a graph through the two databases involving the hubs of the two databases and any other table referred to in the query. This graph will comprise a partial graph exclusively relating to the hub in one database, a second graph exclusively related to the hub of the other database and a partial graph connecting the hub of the first database to the hub of the second database and extending through said two linking tables. It should be noted that the linking tables are not necessarily the hub tables.

Again, the evaluation of the query is analogous to what was described before. One will evaluate the partial query related to the first and second hub, putting junctions between the three partial graphs and evaluating the route between the two hubs in the manner described. It should be noted that in evaluating the graph between the two hubs one can again use the concept of junctions, if there is no direct link between the two hubs. One will, in the case of two hubs, determine an optimum path through the node diagram from one hub to the other and put junctions between certain nodes, thus defining partial queries which are evaluated separately and the results of which are eventually combined with each other to render a partial result for this partial graph connecting the two hubs which is then merged with the results of the remaining two partial graphs relating to respective hubs.

If the query conditions relate only to one database and the second database is only needed to obtain information related to entries meeting said query conditions, one may proceed, as described before for the case of two hubs in one single database, by retrieving the primary keys of the hub of the first database related to the entries of the query conditions, then evaluate the dynamic link to the hub in the other database and, depending on the value of the primary keys of the hub of the second database retrieved by the evaluation of this link, evaluate the partial graph deriving from the hub of said second database.

The above-mentioned concept of splitting a query into several partial queries, each involving a hub, can also be applied to evaluate queries which involve a relational database and another searchable entity which comprises an identifier which allows for identifying a sub-entity and extracting data from said sub-entity, especially a flat file. If a relational database and a flat file are involved in the same query, one can split the initial query in three parts, namely in a part involving the hub and tables related thereto in the relational database, a part that involves extracting information from the flat file and a part that relates to the link between the flat file and the relational database. In a way, one could treat a flat file within the concept of this invention in a node diagram like a hub that has no further tables related thereto.

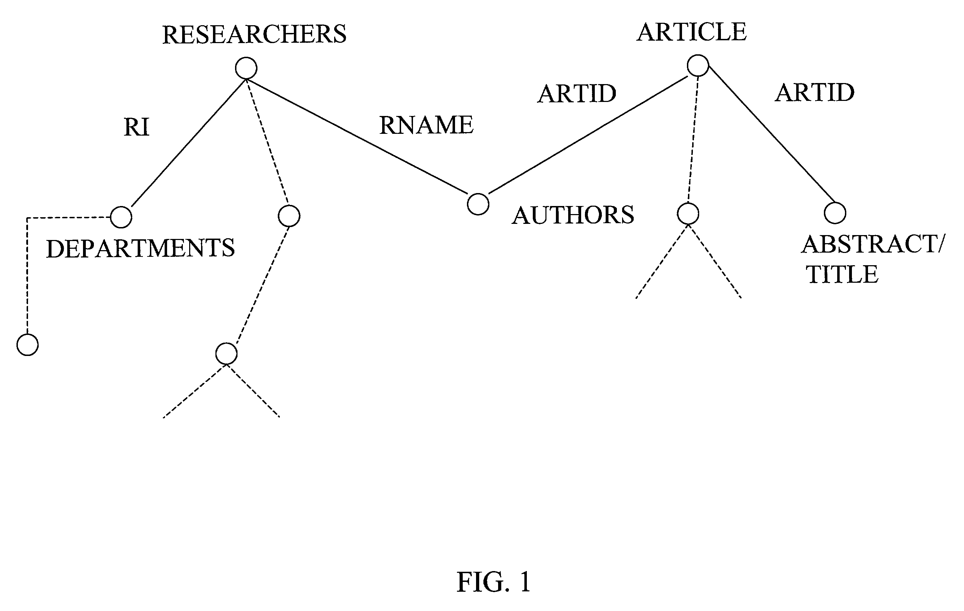

For further illustration of the principles of the present invention, a somewhat simplistic example will be given with reference to the configuration schematically sketched in Fig. 1. Fig. 1 shows a relational database which comprises the tables RESEARCHERS, DEPARTMENTS, ARTICLES, AUTHORS and TITLE/ABSTRACT. There may be further tables which are indicated by dotted lines in Fig. 1. The table RESEARCHERS has columns RNAME and RI (= researchers identifier), the latter defining the primary key for this table. RI forms a link key to the table DEPARTMENTS, RNAME forms a link to column RNAME of table AUTHORS, which also comprises the columns ANAME and ARTID. ARTID in turn forms a link to the table ARTICLE. The table ARTICLE comprises the columns ARTID, JOURNAL YEAR and PAGE. ARTID also forms a link from table ARTICLE to the tables TITLE/ABSTRACT with columns TITLE and ABSTRACT. In this example, the table RESEARCHERS is predefined as the hub table, e.g. because the user of the database is an R & D institution wishing to monitor the research by its employees.

Let us now suppose that a query is submitted for all articles mentioning "insulin" in the abstract and simultaneously requesting to retrieve bibliographic data about the article, the title and the name and the department of the respective author. The system will first look in the table ABSTRACT/TITLE in column ABSTRACT for entries comprising the word "insulin". Subsequently, the system will check whether there is a relation between the table ABSTRACT/TITLE and the hub table RESEARCHERS. As there is, through the chain of tables ABSTRACT/TITLE - ARTICLE - ANAME - RESEARCHERS, the system proceeds with retrieving the keys RI related to entries in the column ABSTRACT in table ABSTRACT/TITLE containing the word "insulin". It turns our that there are two values of RI that match this condition, namely the values 1 and 2 (corresponding to the researchers Smith and Jones).

The system now checks from which tables information is required. These are the tables RESEARCHER, DEPARTMENTS, ARTICLE and ABSTRACT/TITLE. Accordingly, the system will now determine a graph connecting these tables to the hub table RESEARCHERS, as shown by solid lines in Fig. 1. In a first step, the system will then evaluate all entries in the table AUTHOR having the names Smith and Jones (corresponding to the RI 1 and 2; for the sake of simplicity it is presumed that the names are unique identifiers for the researchers) and retrieve related values of ARTID. This result is stored as a first partial result set. One will note that the subsequent tables ARTICLE and ABSTRACT/TITLE were not queried in this step, meaning that a junction was created between the tables AUTHOR and ARTICLE. The key ARTID retrieved in this first step is now used as the input in a subsequent query to retrieve the bibliographic data, the title and the abstract. Reviewing the result of this query one will note that it contains articles 3 and 4, because Smith and Jones are listed as authors for these articles. These articles do, however, not meet the query condition. The system therefore checks the partial result of this second partial query for consistency and removes those parts of the result set which is not consistent with the query condition (abstract contains the word "insulin"). This leaves a result set as shown below with article IDs 1 and 2.

| **RI** | **RNAME** | **ARTID** | **JOURNAL** | **YEAR** | **PAGE** | **TITLE** | **ABSTRACT** |
|---|---|---|---|---|---|---|---|
| 1 | Smith | 1 | Journal X, | 2000 | 193 | Title1 | "....insulin..." |
| 1 | Smith | 2 | Journal Y | 1999 | 295 | Title2 | ".... insulin..." |
| 2 | Jones | 1 | Journal X | 2000 | 193 | Title1 | ".....insulin...." |

One will note that the article ID 1 appears twice, because Smith and Jones are co-authors of article 1.

Having now evaluated the first branch of the graph, the result of which is shown in the result set above, the system now turns to evaluating the second branch comprising the hub RESEARCHERS and the table DEPARTMENTS. As there are only two tables involved in this branch the system retrieves, without the need for a further junction, the information that RI 1 and 2 correspond to Smith and Jones and that Smith is working in department A and Jones is working in department B. This result is now combined with the previous result set so that the entire result set looks as shown below.

| **RI** | **RNAME** | **DEPID** | **ARTID** | **JOURNAL** | **YEAR** | **PAGE** | **TITLE** | **ABSTRACT** |
|---|---|---|---|---|---|---|---|---|
| 1 | Smith | Department A | 1 | Journal X | 2000 | 193 | Title1 | "....insulin..." |
| 1 | Smith | Department A | 2 | Journal Y | 1999 | 295 | Title2 | "..insulin..." |
| 2 | Jones | Department B | 1 | Journal X, | 2000 | 193 | Title1 | "...insulin.." |

The query is now essentially completed. For the sake of simplicity the result was represented as a result table. According to a preferred embodiment, the results of the three partial queries would have been converted into objects which would then have been combined to yield the result to the entire query.

Let us now assume that, in a different setting of the database, both the tables RESEARCHERS and ARTICLE have been chosen as hubs. In this case, for evaluating the same query, the system would establish in a first step that ARTID 1 and 2 match the query condition (abstract contains the word "insulin"). The system would then in a first step evaluate the partial graph comprising the tables ARTICLE and ABSTRACT/TITLE and retrieve the bibliographic data, title and abstract corresponding to articles 1 and 2. In a second step, the system would, starting from the values 1 and 2 of ARTID, determine the related RIs of table RESEARCHERS through the chain of tables ARTICLE - AUTHORS - RESEARCHERS. This could be done in one single query. One could also contemplate to introduce a junction between AUTHORS and RESEARCHERS, i.e. first evaluating a partial query for keys of AUTHORS, which is then used as the input for a further query for values of RI. As a result, one retrieves the values 1 and 2 for RI. In a third partial query involving the hub table RESEARCHERS and the table DEPARTMENTS are retrieved. The result sets of the partial queries are combined, which will result in the same result set for the initial query as shown above.

If one views the chain of tables ARTICLE - AUTHORS - RESEARCHERS as a link between the two hubs, one understands that the same procedure could be applied if the table RESEARCHERS and ARTICLE belonged to different databases or different libraries and were connected through a link between the tables AUTHORS and RESEARCHERS. It should be understood that the link between the databases does not have to be to the hub table of one of the databases or libraries.

In a modification of the above example of two hubs in one database, one could also in a first step determine the values of the primary keys of both hubs meeting the query condition. This would return the RIs 1 and 2 and the ARTIDs 1 and 2, which could then be used to evaluate the two branches of the graph involving the respective hub table and the tables depending therefrom. As a further alternative, one could choose to implement a direct static link between ARTICLES and RESEARCHERS.

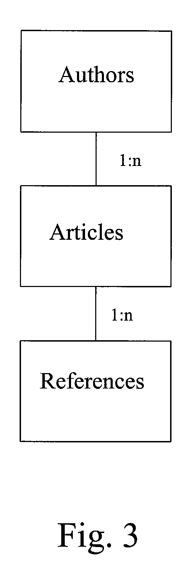

In a further example, the advantage of involving junctions is shown with reference to the database shown in Fig. 3. This database comprises three tables, shown below, with the table "Articles Table" being defined as the hub table and ArticleID as the unique identifier of said hub table.

| **Articles Table** | |
|---|---|
| ArticleID | Title |
| 12 | Title12 |
| 88 | Title88 |
| 754 | Title754 |

| **References Table** | |
|---|---|
| ArticleID | Ref |
| 88 | RefP1 |
| 34 | RefP467 |
| 88 | RefP2 |
| 88 | RefP3 |

| **Authors Table** | |
|---|---|
| ArticleID | Auth |
| 88 | Smith |
| 12 | Klein |
| 88 | Jones |
| 34 | French |

Considering a search for all information related to Article number 88, two cases are demonstrated, namely a case without any junctions and a case with junctions between the hub table (Articles) and each of the related tables (References and Authors)

### Case 1: No Junctions

A single query is constructed and submitted. The result set will have 3 multiplied by 2 = 6 rows

| **Single Result Set Returned** | | | |
|---|---|---|---|
| ArticleID | Title | Ref | Auth |
| 88 | Title88 | RefP1 | Smith |
| 88 | Title88 | RefP1 | Jones |
| 88 | Title88 | RefP2 | Smith |
| 88 | Title88 | RefP2 | Jones |
| 88 | Title88 | RefP3 | Smith |
| 88 | Title88 | RefP3 | Jones |

The redundant data will be ignored during the filtering process and an object constructed from the unique data. In this case, for example, the data "88" and "Title88" will be read only once even though it appears 6 times. The same applies for RefP1, RefP2, RefP3 and the Smith and Jones redundancies. Each unique field is only read once owing to the filtering and object creation algorithm. Expressing the search result as an object or a structure of objects, it is possible to avoid redundancies in the result. This being so, it would, of course, be preferable to avoid redundancies in the first place. This can be done with the concept of junctions.

### Case 2: Both links are junctions

Two queries are constructed and submitted either sequentially or in parallel. Two result sets are returned which are shown below.

| **Result Set 1** | | |
|---|---|---|
| ArticleID | Title | Ref |
| 88 | Title88 | RefP1 |
| 88 | Title88 | RefP2 |
| 88 | Title88 | RefP3 |

| **Result Set 2** | | |
|---|---|---|
| ArticleID | Title | Ref |
| 88 | Title88 | Smith |
| 88 | Title88 | Jones |

The redundant data is ignored (only "88" and "Title88" in this case). An object is constructed from these two result sets. This object is equivalent to the object constructed in Case 1.

One will note that with the single query, the result set is larger and contains more redundant information. The number of returned rows will dramatically rise as the number of tables (with other than 1-1 relationships) increases. Complex relationships are the usual situation in most real-world databases.

It should be understood that the above examples are merely for the purpose of illustration and that the invention is in no way limited to these examples.

The features of the invention disclosed in the specification, the claims and the drawings can, both singly, as well as in any arbitrary combination, be material for the realization of the invention in its various embodiments.

## Claims

1. Method of evaluating a query involving at least one relational database comprising a relational database management system (RDBMS), said query relating to at least one table of said relational database,
said method comprising
determining a table of said relational database as a gateway table for evaluating said query,
retrieving one or more unique identifiers of said gateway table related to one or more entries in a table to be queried,
retrieving information from one ore more tables to be queried related to said retrieved unique identifiers of said gateway table,
providing a result to said query.

2. Method according to claim 1, wherein said relational database comprises one or more predetermined hub tables and said query relates to at least one table of said relational database and wherein said method comprises:
retrieving one or more unique identifiers of a hub table related to one or more entries in a table to be queried,
retrieving information from tables to be queried related to said retrieved unique identifiers of said hub table,
providing a result to said query.

3. Method according to one of claims 1 or 2, wherein at least one library is defined on one or more of said databases, said library consisting of tables linked to each other and having exactly one table defined as a hub table.

4. Method according to one of claims 1 to 3, wherein said query is for complete sets of related entries of said relational database or of a library or for parts of such complete sets of related entries and comprises one or more query conditions related to said database or library, wherein said method comprises:
- identifying a gateway table related to entries specified in a query condition,
- identifying one or more unique identifiers of said gateway table related to said entries conforming to query conditions,
- retrieving complete sets of related entries or part thereof which are related to said unique identifiers of said gateway table.

5. Method according to one of claims 2 to 4, wherein said query involves at least a second searchable entity outside said database or outside a library involved in said query, said second entity comprising sub-entity each having at least one identifier uniquely identifying said sub-entities, and wherein said method comprises:
- retrieving one or more identifiers of sub-entities of said second searchable entity related to said query,
- retrieving one or more unique identifiers of a hub table of said relational database or library related to said retrieved identifiers of said sub-entities,
- retrieving sets of related entries or predetermined parts thereof related to said retrieved unique identifiers of said hub table,
- retrieving information from a sub-entity identified by a retrieved identifier in said second entity,
- combining the retrieved information from said second searchable entity and said data base or library into a result.

6. Method according to one of claims 2 to 5, wherein said query involves at least a second searchable entity outside said database or outside a library involved in said query and comprising sub-entities, each sub-entity having at least one identifier uniquely specifying said sub-entity, and wherein said method comprises:
- retrieving one or more unique identifiers of a hub table of said database or library related to entries related to said query,
- retrieving identifiers of sub-entities of said second searchable entity related to said retrieved unique identifiers of said hub table,
- retrieving sets of related entries or predetermined parts thereof related to said retrieved unique identifiers of said hub table,
- retrieving information from said sub-entities identified by identifiers retrieved in said second searchable entity,
- combining the retrieved information from said second entity and said data base or library into a result.

7. Method according to one of claims 5 or 6, wherein said second searchable entity is a relational database or a library and said identifier is a unique identifier of a hub table in said relational database or library.

8. Method according to one of claims 5 or 6, wherein said second searchable entity is a collection of flat files with the sub-entities being flat files in this collection.

9. Method according to one of claims 4 to 8, wherein said step of retrieving a relation between identifiers of said second searchable entity and unique identifiers of hubs of said database or library comprises the step of discarding combinations of identifiers of hubs with identifiers of said second searchable entity which are not consistent with the query conditions and retrieving only such additional information related to an identifier which is comprised in a combination of identifiers consistent with the selection parameters.

10. Method according to one of claims 2 to 9, wherein the query relates to tables related to at least two hub tables, wherein said method comprises:
- retrieving one or unique identifiers of a hub table or hub tables, said identifier being related to entries satisfying query conditions in tables related to the respective hub,
- retrieving unique identifiers of the respective other hub or hubs related to said retrieved unique identifiers related to entries satisfying the query conditions,
- retrieving sets of related entries or predetermined parts thereof, which are related to said retrieved unique identifiers of said hubs according to the query,
- combining the retrieved information related to said hubs into a result.

11. Method according to claim 10, wherein said step of retrieving a relation between unique identifiers of said hub tables comprises the step of discarding combinations of unique identifiers of hub tables which are not consistent with the query conditions and retrieving only such additional information related to a unique identifier which is comprised in combinations of unique identifiers consistent with the search parameters.

12. Method according to claim 10 or 11, wherein at least one of said hubs is the hub of a library and the query relates to said library.

13. Method according to claim 12, wherein the query relates to two libraries and said hubs are hubs of two libraries.

14. Method according to one of claims 10 to 13, wherein said two hubs are hubs within the same relational database.

15. Method according to one of claims 4 to 14, wherein the step of retrieving unique identifiers of a hub table and/or identifiers of a searchable entity which are related to another unique identifier of a hub table and/or identifier of a searchable entity is performed on the basis of pre-established relations between identifiers of said entities.

16. Method according to one of claims 4 to 14, wherein the step of retrieving unique identifiers of a hub table and/or identifiers of a searchable entity which are related to another unique identifier of a hub table and/or identifier of a searchable entity is performed dynamically during the execution of the query.

17. Method according to one of claims 1 to 16, wherein in performing said step of retrieving information related to a unique identifier of said gateway table, selected tables are queried which, in a graphical representation of the database wherein the tables are represented as nodes and links between the tables are represented as lines between the nodes, form a connected graph connecting the gateway table to tables referred to in the initial query.

18. Method according to claim 17, wherein said step of querying tables on said graph comprises performing consecutive partial queries, wherein a result of a previous query is used as input for a later query, a first of said partial queries involving the gateway table and a query other than the first query relating to a table referred to in the initial query.

19. Method according to claim 18, wherein said result of said previous query comprises the value of a foreign key of a table involved in said later query and wherein said value of said foreign key is used as input for said later query.

20. Method according to claim 18 or 19, wherein the result of said partial queries is stored as an object or objects.

21. Method according to one of claims 18 to 20, wherein after each partial query a redundancy check and/or a check for consistency is carried out in the respective result and the result purged of redundancies and/or inconsistencies is stored.

22. Method according to claim 21, wherein said redundancy check is carried out in creating said object comprising the result of said partial query or directly on said object after creation of the same.

23. Method according to one of claims 18 to 22, wherein each partial query involves a table or a plurality of tables linked to each other and wherein each partial query other than the first has as input previously established values of link keys, said link keys linking said table or one or more of said plurality of tables to another table not involved in said partial query.

24. Method according to one of claims 18 to 23, wherein said graph comprises at least one branch node having links to at least two other nodes and wherein tables referred to in the initial query are related to separate branches deriving from said branch node, wherein a partial query is carried out involving the table corresponding to said branch node (branch table) and wherein at least one partial query is carried out for one or more tables contained in each branch which has the result of the partial query involving the branch table as an input.

25. Method according to one of claims 18 to 24 comprising the steps of
- identifying the hub table or hub tables related to tables referred to in the initial query,
- determining in said graphical representation of said database, at least for one hub table, an optimum graph connecting said hub to all tables which are related to said hub and which are referred to in the initial query,
- performing consecutive partial queries involving tables which are consecutive to each other with regard to said optimum graph.

26. Method according to one of claims 1 to 25, wherein said step of retrieving unique identifiers of said gateway table comprises:
- determining a table that is referred to in the initial query,
- determining, in a graphical representation of said database, wherein tables are represented as nodes and links between tables as lines between the nodes, a gateway table connected to said table,
- querying said database for one or more indices of the gateway table which are related to said table.

27. Method according to claim 26, wherein one or more specific entries of said table are implied by a query condition and said database is queried for indices of said gateway table which are related to said entry or entries.

28. Method according to claim 26 or 27, wherein in said graphical representation, a path from said table to said gateway table is established and said query for said indices is performed by querying all tables corresponding to nodes in said graph for the values of link keys between the tables in said graph, starting from the table referred to in the query and, given the case, certain entries thereof.

29. Method according to claim 28, wherein said path is selected as a shortest path between said table and said gateway table according to a predetermined metric.

30. Method according to claim 28 or 29, wherein said path is part of or identical to the graph for determining partial queries for retrieving additional information from tables related to said gateway table.

31. Method according to one of claims 26 to 30, comprising the step of:
- determining a unique identifier for one or more rows of the gateway table related to said indices, if an index or a group of indices related to the same row of the gateway table determined by said step of querying the database does not uniquely identify a row of said gateway table.

32. Method according to one of claims 1 to 31, wherein partial queries used for evaluating the initial query are at least partially created dynamically during the process of said evaluation.

33. Method according to one of claims 1 to 32, wherein said result is represented in an object oriented representation.

34. Method according to claim 33, wherein the result of said initial query is expressed as an object derived by means of object-relational mapping.

35. Method according to one of claims 1 to 34, wherein said evaluation of said query is performed under the control of an object manager, said object manager comprising a sequence of commands to be executed by a computer system.

36. Method according to claim 35, wherein said object manager handles an object which represents the schema or part of a schema of one or more databases to be queried.

37. Method according to claim 35 or 36, wherein said object manager defines classes which are dynamically created and instantiated.

38. Data processing system for controlling the evaluation of a query involving a relational database comprising a relational database management system (RDBMS), said query relating to at least one table of said relational database, comprising:
- means for determining a table as a gateway table for evaluating said query,
- means for establishing a relation between a table or tables to be queried and a gateway table,
- means for causing the RDBMS to retrieve one or more unique identifiers of said gateway table related to one or more entries in a table to be queried,
- means for causing the RDBMS to retrieve information from tables to be queried related to said retrieved unique identifiers of said entry,
- means for providing or causing to be provided a result to said query.

39. Data processing system according to claim 38, comprising means for setting certain tables in said relational database as predetermined gateway tables for queries to be evaluated.

40. Data processing system according to claim 38 or 39, comprising means for controlling the execution of a method according to one of claims 1 to 37 by a data processing system or data processing systems.

41. Computer program causing a computer or computer system, when executed thereon, to perform the steps of a method according to one of claims 1 to 37.

42. A computer readable storage medium, comprising a program according to claim 41.
